(19) Europäisches Patentamt
European Patent Office
Office européen des brevets

(11) Publication number: **0 103 030**
**A1**

(12) **EUROPEAN PATENT APPLICATION**

(21) Application number: **82108107.2**

(22) Date of filing: **02.09.82**

(51) Int. Cl.³: **C 07 D 491/10**, A 61 K 31/415
// C07D231/54 ,(C07D491/10, 311/00, 231/00)

(43) Date of publication of application: **21.03.84**
**Bulletin 84/12**

(84) Designated Contracting States: **BE CH DE FR GB IT LI LU NL SE**

(71) Applicant: **STERLING DRUG INC., 90 Park Avenue, New York New York (US)**

(72) Inventor: **Bell, Malcolm Rice, R.D. 1 Box 156 A, East Greenbush New York (US)**
Inventor: **Herrmann, John Lawrence, Jr., 46 Williams Street, Kinderhook New York (US)**

(74) Representative: **Baillie, Iain Cameron et al, c/o Ladas & Parry Isartorplatz 5, D-8000 München 2 (DE)**

(54) **Polycyclic fused pyrazole compounds, useful as anti-inflammatory agents, and preparation thereof.**

(57) The compounds of formula

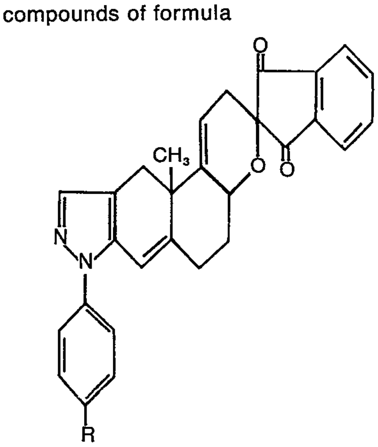

where R is hydrogen or fluorine possess glucocorticoid activity, and are prepared by reacting a compound of the formula

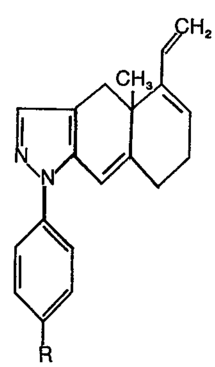

with 1,2,3-indantrione.

EP 0 103 030 A1

The present invention relates to novel polycyclic fused pyrazole compounds, their use as anti-inflammatory agents, and a method of preparation thereof.

Typical glucocorticoid activity is rarely found in structures which do not possess an intact steroid nucleus. Such activity is found in naturally occurring steroids such as cortisone, hydrocortisone and aldosterone, as well as numerous synthetic modifications thereof, all containing the intact steroid nucleus. An example of a synthetic cortical steroid having high activity is a fluorophenylpyrazole derivative reported by Fried et al., J. Am. Chem. Soc. 85, 236 (1963), having the structure

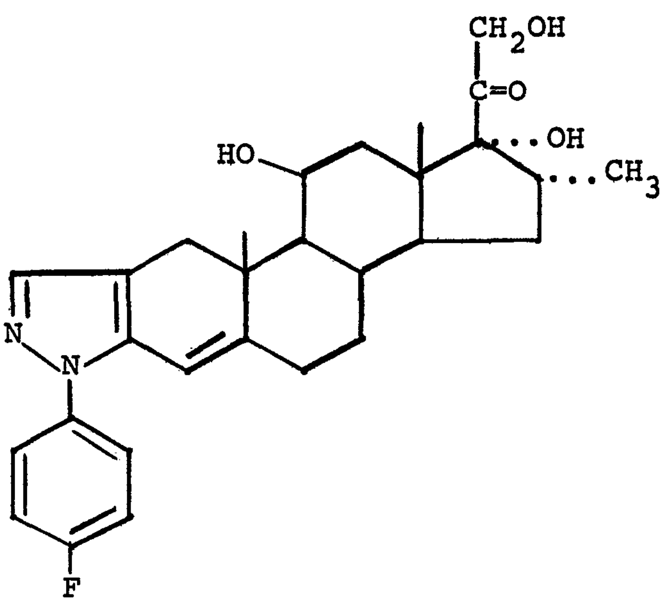

The present invention relates to compounds having the formula:

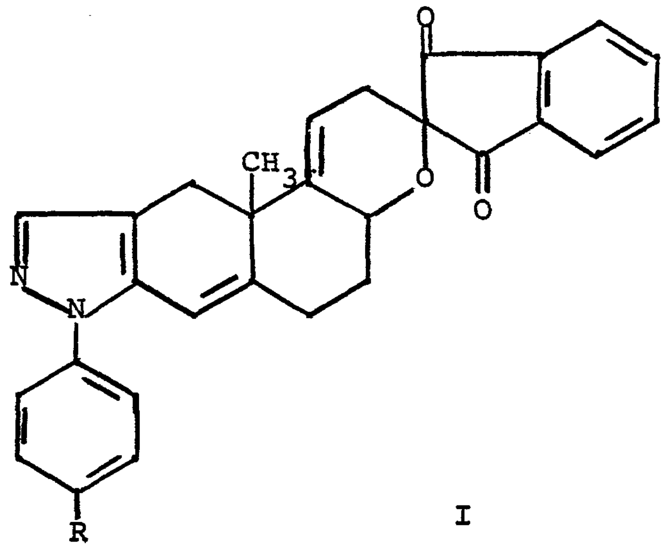

I

where R is hydrogen or fluorine.

A pharmaceutical composition for treating inflammation in mammals comprises an anti-inflammatorily effective amount of a compound of Formula I and a pharmaceutically acceptable carrier. One can reduce inflammation in a mammal by administering to said mammal an anti-inflammatorily effective amount of a compound of Formula I.

One can prepare a compound of Formula I by reacting a compound of the formula:

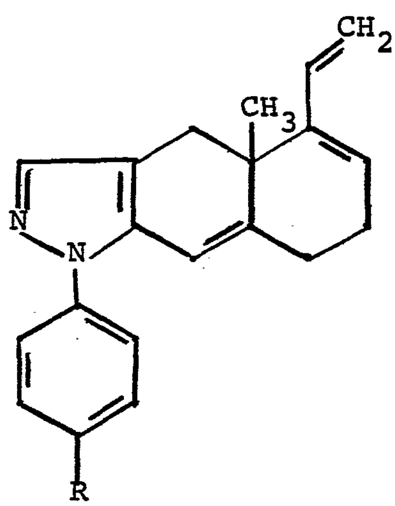

II

with 1,2,3-indantrione.

The intermediates of Formula II are prepared from a known starting material, 5-ethenyl-4,4a,7,8-tetrahydro-4a-methyl-2(3H)-naphthalenone U.S. Patent 4,157,349, in accordance with the following reactions:

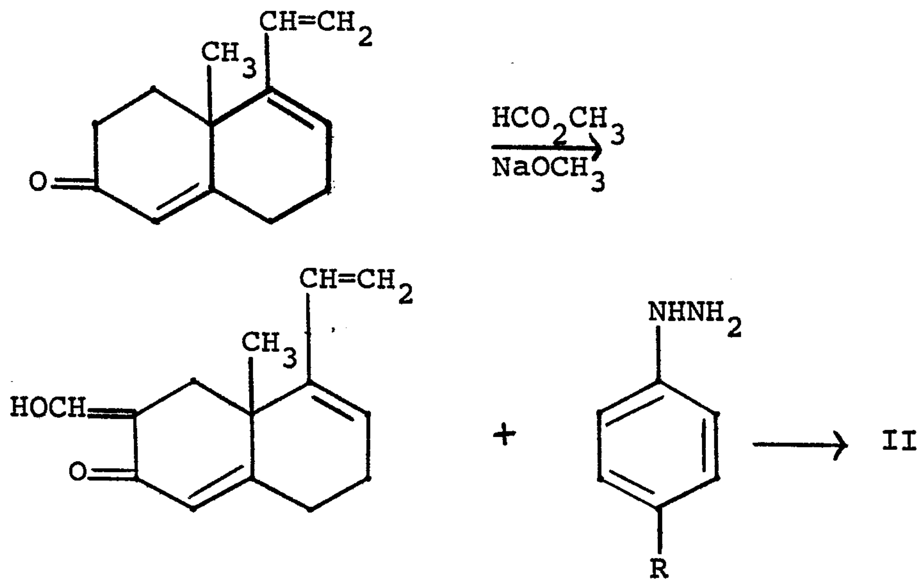


The trienone starting material is reacted with methyl formate in the presence of sodium methoxide in an inert solvent such as tetrahydrofuran to afford 5-ethenyl-3-hydroxymethylene-4,4a,7,8-tetrahydro-4a-methyl-2(3H)-naphthalenone, and the latter is then reacted with phenylhydrazine or 4-fluorophenylhydrazine or an acid-addition salt thereof in the presence of acetic acid to give the compound of Formula II.

The reaction of a compound of Formula II with 1,2,3-indantrione takes place by heating the reactants in an inert solvent at a temperature between about 50° and 150°C.

The compounds of Formula I exhibit an endocrinological profile characteristic of compounds possessing glucocorticoid properties and systemic and/or topical anti-inflammatory activity; cf. R.H. Silber. The Biology of Anti-inflammatory Steroids, Annals of the New York Academy of Sciences, Vol. 82, Art. 4, pp. 821-828.

When the compounds of Formula I are administered orally to rats they cause a significant depression in thymus weight, adrenal weight and body weight gain without a change in food consumption.

The compound of Formula I where R is F has also been found to possess oral glucocorticoid activity by the

liver glycogen deposition test and anti-inflammatory activity by the α-tocopherol pouch test in rats.

The test procedures used to determine the biological activities of the compounds of the invention were carried out as follows:

Endocrine Profile: Mature female rats with an average body weight of 202 g and a body weight range of 15 g or less were medicated orally with test compound for 2 weeks. The test compound was prepared as a solution or suspension in 1% gum tragacanth or 0.75% methyl cellulose. On the day following the last medication, the rats were killed and the thymus and adrenal of each rat were removed, cleaned, and weighed. Body weights and food consumptions were also recorded.

Anti-inflammatory Activity (α-tocopherol pouch test): Male rats which weighed 120 g were selected for testing. A rapid subcutaneous injection of 25 mL of air was made between the scapulae of each rat. This resulted in the establishment of an airfilled pouch into which 0.5 mL of dl-α-tocopherol was injected. The test compound was administered in daily oral doses for 7 days beginning on the day of pouch formation. The compound to be tested was suspended in 1% gum tragacanth. Twenty-four hours after the last medication, the pouches were dissected free, and the fluid volume was measured. The inhibition of liquid exudate is a measure of the anti-inflammatory activity.

Glycogenic Activity: Mature male rats were bilaterally adrenalectomized 5 days prior to the test. These rats were medicated orally with the test compound for 5 days. Seven hours after the last medication, the rats (which have been fasted overnight) were anesthetized with sodium pentobarbital and a portion of one lobe of the liver was removed and frozen on dry ice for subsequent glycogen determination.

The compounds of the invention can be formulated for topical application by solution or dispersion in a

conventional pharmaceutically acceptable liquid, cream or ointment base. The effective ingredient is preferably present in a concentration of 0.01% to 5.0% by weight.

The compounds of the invention can be formulated for oral administration in tablet or capsule form with conventional excipients. The active ingredient is preferably present in an amount of 1 mg to 100 mg per unit dosage form.

The following examples will further illustrate the invention.

Example 1

(a) 5-Ethenyl-3-hydroxymethylene-4,4a,7,8-tetrahydro-4a-methyl-2(3H)-naphthalenone.

A solution of 50.0 g (0.265 mol) of 5-ethenyl-4,4a,7,8-tetrahydro-4a-methyl-2(3H)-naphthalenone in 350 mL of tetrahydrofuran was cooled to -5°C. in an ice-methanol bath and stirred under nitrogen while 57.2 g (1.06 mol) of sodium methoxide was added. The resulting mixture was stirred for 30 min at -5°C. and then a solution of 114 mL (1.85 mol) of methyl formate in 100 mL of tetrahydrofuran was added slowly. The mixture was stirred overnight at room temperature and then poured onto a mixture of ice-water (1500 mL) and 6N hydrochloric acid (265 mL). The product was extracted with ether and the combined extracts were washed with water. The dried extract was dried over anhydrous magnesium sulfate and concentrated *in vacuo* to afford an oil. This oil was triturated with hexane (4 x 250 mL) and the combined triturates were dried over magnesium sulfate and concentrated *in vacuo* to afford 55.37 g of a red oil, consisting essentially of the above-entitled compound as established by proton NMR (PMR) spectral data.

(b) 1-Ethenyl-6-(4-fluorophenyl)-3,4,9,9a-tetrahydro-9a-methyl-6H—naphtho[2,3-c]pyrazole (II; R = F).

4-Fluorophenylhydrazine hydrochloride (45.85 g, 0.282 mol) and sodium acetate (23.14 g, 0.282 mol) were added to a solution of 55.37 g (0.256 mol) of the product

obtained in part (a) above in 225 mL of glacial acetic acid. The mixture was stirred overnight at room temperature and then concentrated in vacuo to afford a semi-solid. This material was suspended in ether (1 L) and filtered to remove sodium chloride. The ether filtrate was washed with water (4 x 250 mL), saturated sodium bicarbonate (until weakly basic) and saturated sodium chloride (100 mL). The extract was dried over anhydrous magnesium sulfate, decolorized with charcoal and concentrated in vacuo to afford an oil. This oil was triturated with 1:2 ether-hexane (3 x 750 mL) to afford 69.58 g of a dark brown oil. An analytical sample was prepared by using high-performance liquid chromatography with 1:3 ether-hexane as solvent. The resulting yellow oil was triturated with pentane to afford 1-ethenyl-6-(4-fluorophenyl)-3,4,9,9a-tetrahydro-9a-methyl-6H—naphtho[2,3-c]pyrazole (II; R = F) as a yellow solid, m.p. 70-72°C. with a consistent PMR spectrum.

(c) 8'-(4-Fluorophenyl)-2',3',5',6',11',11a'-hexahydro-11a'-methylspiro-2H-indene[2,3']-3H-pyrazolo[4",5":7',6']-naphtho-[2,1-b]pyran-1,3-dione (I; R = F).

A mixture of 15.3 g (0.05 mol) of II (R = F) (part b above) and 9.8 g (0.055 mol) of 1,2,3-indantrione monohydrate in 150 mL of xylene was refluxed for 2 hours. The cooled reaction mixture was filtered through silica gel and concentrated in vacuo. The residue was crystallized from ethanol to afford 7.19 g of I (R = F) as a tan solid, m.p. 210-211°C. The proton NMR spectrum (PMR) was consistent with the assigned structure.

In the endocrine profile determination, Compound I (R = F) at a dose level of 1 mg/kg caused a 61% reduction in thymus weight, 56% reduction in adrenal weight and 129% reduction in body weight gain as compared with the controls. In the α-tocopherol pouch test, Compound I (R = F) was active with $ED_{50}$ = 10 mg/kg. In the glycogenic activity test, Compound I (R = F) at a dose level of 9 mg/kg/day x 5 produced a liver glycogen deposition value of 21.8 ± 4.4 mg/g of tissue as compared to 2.2 ± 0.04 mg/g for the

vehicle (1% gum tragacanth) alone.

Example 2

(a) 1-Ethenyl-6-phenyl-3,4,9,9a-tetrahydro-9a-methyl-6H-naphtho[2,3-c]pyrazole (II; R = H) was prepared according to the procedure of Example 1, part (b), while substituting a molar equivalent amount of phenylhydrazine hydrochloride for the 4-fluorophenylhydrazine hydrochloride of that example. The product was characterized by its PMR spectrum.

(b) 2',3',5',6',11',11a'-Hexahydro-11a'-methyl-8'-phenylspiro 2H-indene[2,3']-3H-pyrazolo[4",5":7',6']naphtho[2,1-b] pyran-1,3-dione (I; R = H).

A mixture of 14.42 g (0.05 mol) of II (R = H) (part a above) and 9.88 g (0.06 mol) of 1,2,3-indantrione monohydrate in 150 mL xylene was refluxed for 2 hours, then allowed to stand at room temperature for 55 hours and filtered to afford a green solid. This green solid was dissolved in methylene dichloride, filtered through silica gel and the solvent removed *in vacuo*. The residue was triturated with ether to afford 10.95 g of I (R = H) as a yellow solid, m.p. 209-210°C. The PMR spectrum was consistent with the assigned structure.

In the endocrine profile determination, Compound I (R = H) at a dose level of 5 mg/kg caused a 51% reduction in thymus weight, 38% reduction in adrenal weight, and 62% reduction in body weight gain as compared with the controls. Compound I (R = H) was inactive in the α-tocopherol pouch test at a dose level of 100 mg/kg.

# CLAIMS

1. A compound of the Formula I (herein) where R is hydrogen or fluorine.

2. 8'-(4-Fluorophenyl)-2',3',5',6',11',11a'-hexahydro-11a'-methylspiro-2H-indene[2,3']-3H-pyrazolo[4",5":7',6']naphtho[2,1-b]pyran-1,3-dione, according to claim 1.

3. 2',3',5',6',11',11a'-Hexahydro-11a'-methyl-8'-phenylspiro-2H-indene[2,3']-3H-pyrazolo[4",5":7',6']naphtho[2,1-b]pyran-1,3-dione, according to claim 1.

4. A process for preparing a compound according to claim 1, which comprises reacting 1-ethenyl-6-(4-R-phenyl)-3,4,9,9a-tetrahydro-9a-methyl-6H-naphtho[2,3-c]pyrazole with 1,2,3-indantrione.

5. A process according to claim 4, wherein R is fluorine.

6. A pharmaceutical composition for treating inflammation in mammals which comprises an anti-inflammatorily effective amount of a compound according to any one of claims 1-3 and a pharmaceutically acceptable carrier.

European Patent Office

# EUROPEAN SEARCH REPORT

0103030

Application number

EP 82 10 8107

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| A | EP-A-0 058 841 (STERLING) * Claims 1,10 * ----- | 1,6 | C 07 D 491/10<br>A 61 K 31/415//<br>C 07 D 231/54<br>(C 07 D 491/10<br>C 07 D 311/00<br>C 07 D 231/00 ) |
| | | | TECHNICAL FIELDS SEARCHED (Int. Cl. 3) |
| | | | C 07 D 491/00<br>A 61 K 31/00 |

The present search report has been drawn up for all claims

| Place of search | Date of completion of the search | Examiner |
|---|---|---|
| THE HAGUE | 22-04-1983 | ALFARO I. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document